# DEMANDE DE BREVET EUROPEEN

(11) **EP 2 198 826 A1**
(43) Date de publication de la demande: **23.06.2010**
(21) Numéro de dépôt: 09179534.4
(22) Date de dépôt: 16.12.2009
(51) Int. Cl.: A61K 8/02, A61K 8/19, A61K 8/891, A61Q 1/12, A45D 34/04, A45D 40/18, A45D 40/26

(54) **Procédé de maquillage et ensemble cosmetique pour la mise en oeuvre d'un tel procédé.**

(30) Priorité: 18.12.2008 FR 0858767; 08.01.2009 US 193922 P
(71) Demandeur: L'Oréal, 75008 Paris (FR)
(72) Inventeur: Blin, Xavier, 75015, Paris (FR); Claude-Foly, Coralie, 60550, Verneuil En Halatte (FR); Beaumard, Sophie, 94800, Villejuif (FR); Arnaud, Pascal, 94240, L'Haye-Les-Roses (FR); Blondeau, Françoise, 94600, Choisy Le Roi (FR)
(74) Mandataire: Tanty, François

(57) **Abrégé**

Procédé de maquillage et/ou de soin de la peau, notamment pour lisser le microrelief cutané, comportant les étapes consistant à :
- appliquer une composition cosmétique sous forme de poudre libre au moyen d'un applicateur vibrant, la composition comportant :
- un matériau particulaire lamellaire en une proportion massique supérieure ou égale à 5 % par rapport au poids total de la composition,
- entre 0,1 et 7 % en masse d'un liant hydrocarboné, siliconé et/ou fluoré.

## Description

La présente invention concerne le maquillage et/ou le soin de la peau.

L'utilisation des poudres libres notamment dans le maquillage du teint est très courante.

Ces poudres ont pour fonction d'apporter de la couleur, de la matité et d'améliorer la tenue du fond de teint.

Cependant leur application est souvent problématique en raison de leur caractère pulvérulent. Il est, en effet, facile de salir ses vêtements lorsqu'on les applique.

C'est la raison pour laquelle beaucoup de recherches ont été entreprises pour trouver des applicateurs qui minimisent ces inconvénients et facilitent l'application.

Les applicateurs sous la forme de houppette ou d'éponge ne permettent pas de limiter le caractère volatil de la poudre car celle-ci adhère relativement peu à l'applicateur.

Plus récemment, des pinceaux ont été proposés, mais la gestuelle au cours de l'application et le manque d'adhérence de la poudre sur les fibres du pinceau ne permettent pas d'obtenir une application complètement satisfaisante.

Il reste donc nécessaire de rechercher de nouveaux ensembles cosmétiques et procédés de maquillage qui permettent d'éviter les inconvénients cités ci-dessus.

L'invention a pour objet, selon l'un de ses aspects, un procédé de maquillage et/ou de soin de la peau, notamment pour lisser le microrelief cutané, comportant les étapes consistant à :
- appliquer une composition cosmétique sous forme de poudre libre au moyen d'un applicateur vibrant, la composition comportant :
   - un matériau particulaire lamellaire en une proportion massique supérieure ou égale à 5 % par rapport au poids total de la composition,
   - entre 0,1 et 7 % en masse d'un liant hydrocarboné, siliconé et/ou fluoré.
En particulier, le procédé de maquillage et/ou de soin selon l'invention est destiné à lisser les imperfections cutanées de reliefs.

De manière inattendue, l'utilisation d'un applicateur vibrant associé à la poudre libre telle que définie ci-dessus permet d'obtenir un résultat de maquillage et/ou de soin plus lisse, plus uniforme et moins poudreux qu'avec un autre applicateur.

Ce résultat est particulièrement visible sur les femmes qui possèdent des imperfections cutanées de reliefs comme des pores dilatés.

Par ailleurs, l'utilisation d'un applicateur vibrant permet de réduire le caractère volatil de la poudre lors de l'application, diminuant ainsi les risques de se salir.

L'application de vibrations sur la peau permet en outre de masser l'épiderme en vue d'obtenir une sensation de bien-être au niveau de la zone d'application. L'application desdites vibrations peut induire une réponse biologique au niveau des cellules de l'épiderme et/ou du derme, *via* la stimulation de mécanorécepteurs (ex : intégrines), et permettre ainsi d'améliorer l'épaisseur de la peau et/ou améliorer l'éclat du teint et/ou améliorer les propriétés mécaniques de la peau (fermeté, élasticité, tonicité).

Des applicateurs vibrants sont déjà connus.

Le brevet FR 1 223 254 décrit un procédé de répartition sur la peau de produits de maquillage à l'aide d'un applicateur vibrant.

Les demandes FR 2 882 506 et WO 2006/090343 décrivent différents types d'applicateurs vibrants permettant l'application de produits de maquillage.

La demande EP 1 842 520 décrit un applicateur vibrant comportant un amortisseur qui atténue la transmission de la vibration à la main de l'utilisateur.

La demande FR 2 904 923 décrit un dispositif vibrant et un procédé de maquillage utilisant un tel dispositif, le dispositif comportant des moyens de fixation amovibles à un doigt. Cette demande évoque la possibilité d'appliquer une poudre libre.

Dans un exemple de mise en oeuvre de la présente invention, la poudre peut être prélevée sans soumettre l'applicateur à des vibrations, en amenant l'applicateur au contact de la composition contenue dans un récipient. L'applicateur peut être soumis à des vibrations après le prélèvement, alors que l'applicateur est encore au-dessus du récipient, de façon à éliminer un éventuel surplus de composition sur l'applicateur, ce surplus retombant dans le récipient. En variante, l'applicateur peut comprendre une réserve interne de composition qui est délivrée lors de l'application.

La poudre peut être appliquée sur la peau nue ou maquillée. En particulier, la poudre est appliquée sur la peau nue.

L'invention a encore pour objet, selon un autre de ses aspects, un ensemble cosmétique, comportant :
- un récipient contenant une composition cosmétique sous forme de poudre libre, comportant :
- un matériau particulaire lamellaire en une proportion supérieure ou égale à 5 % en poids par rapport au poids total de la composition,
- un liant hydrocarboné, siliconé et/ou fluoré,
- un applicateur,
- une source vibrante pour soumettre l'applicateur à des vibrations au moins au moment de l'application de la composition sur la peau et/ou du prélèvement de la composition.

L'applicateur peut comporter une mousse, un flocage, un pinceau, un tissé ou un non tissé, définissant une surface d'application destinée à venir au contact de la peau.

L'applicateur peut se loger dans le récipient quand celui-ci est fermé.

### Source vibrante

Conformément à l'invention, la surface d'application est soumise à des vibrations, lesquelles proviennent d'une source vibrante.

La source vibrante pouvant convenir, selon l'invention, produit des vibrations qui peuvent être obtenues de diverses manières, notamment par voie mécanique, électronique ou électromécanique.

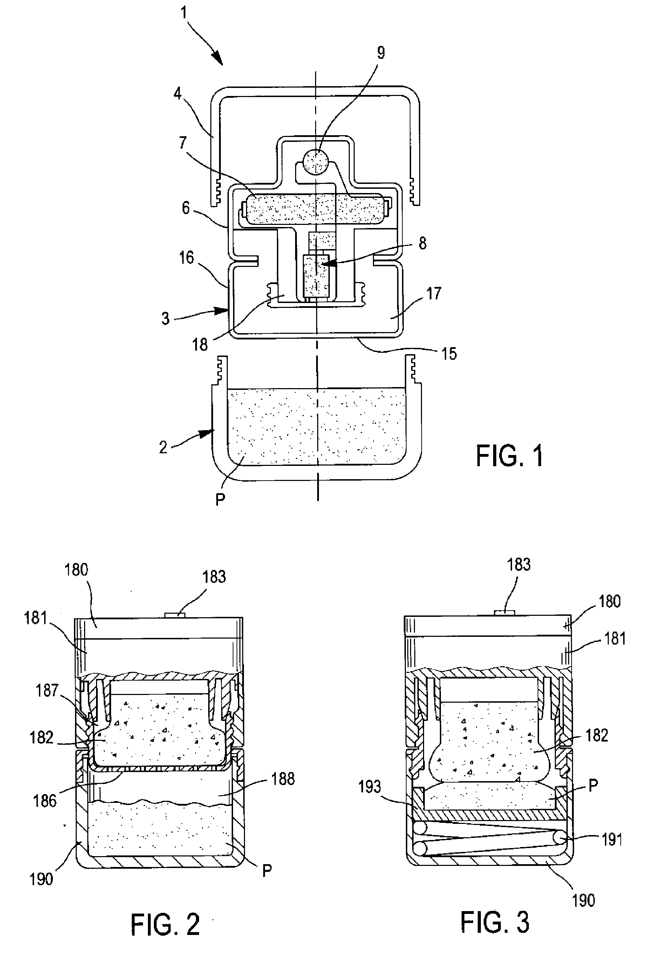

Le dispositif de conditionnement et d'application représenté sur la figure 1 et décrit plus loin comporte une source vibrante permettant de produire des vibrations au prélèvement et à l'application, sur une surface d'application destinée à venir au contact des matières kératiniques lors de l'utilisation du dispositif.

D'une manière générale, la fréquence des vibrations peut être comprise entre 5 Hz et 10 kHz, mieux 100 Hz et 5000 Hz. Selon un mode particulier, la fréquence des vibrations est comprise entre 100 Hz et 1000 Hz, et notamment de 100 Hz à 300 Hz.

La source vibrante peut comporter un vibreur composé d'un moteur et d'une masselotte entraînée en rotation par le moteur et dont le centre de gravité est excentré par rapport à l'axe de rotation. Le moteur peut être alimenté électriquement par une source d'énergie telle que par exemple une pile bâton, reliée électriquement au moteur par un interrupteur.

La source vibrante peut comporter un vibreur autre qu'un moteur électrique entraînant en rotation une masselotte. La source vibrante peut notamment comporter tout système électromécanique, piézoélectrique, pneumatique, hydraulique, mécanique, électronique ou électromécanique capable de produire des vibrations.

La source vibrante peut comporter des moyens de contrôle des vibrations autres qu'un simple interrupteur de marche/arrêt et notamment comporter des moyens de contrôle, mécaniques ou électroniques, permettant de régler l'amplitude et/ou la fréquence des vibrations. Les moyens de contrôle peuvent par exemple comporter un potentiomètre ou un commutateur, rotatif ou linéaire, permettant de sélectionner au moins deux vitesses de rotation du moteur électrique dans le cas où le vibreur comporte un tel moteur.

La source vibrante peut comporter plus d'un vibreur et par exemple deux vibreurs agencés pour produire des oscillations selon des directions différentes. Dans ce cas, l'applicateur peut également comporter par exemple un sélecteur permettant de sélectionner le ou les vibreurs que l'on souhaite mettre en fonctionnement.

La source vibrante peut, le cas échéant, être orientée par l'utilisateur de façon à faire vibrer l'élément d'application définissant la surface d'application vibrante avec des vibrations d'orientation voulue.

La source vibrante peut comporter une source d'énergie qui peut être autre qu'une pile et notamment comporter un ou plusieurs accumulateurs ou condensateurs. La source vibrante peut être agencée de manière à pouvoir être rechargée en électricité en étant reposée sur un socle. Le cas échéant, la source vibrante peut être alimentée par le secteur par l'intermédiaire d'un transformateur éventuel.

La source vibrante peut être montée de multiples manières dans un logement correspondant de l'applicateur ou du dispositif de conditionnement et de distribution, et le montage de la source vibrante est par exemple conçu de manière à favoriser le transfert de vibrations vers la surface d'application ou vers la surface de préhension.

La source vibrante est par exemple disposée dans l'applicateur avec interposition de moyens d'amortissement élastiques entre le boîtier de l'applicateur et la source vibrante. Ces moyens d'amortissement comportent par exemple un joint en élastomère.

La source vibrante peut encore être portée par le doigt de l'utilisateur, comme décrit dans la demande FR 2 904 923.

### Exemples d'ensembles cosmétiques pour la mise en oeuvre de l'invention

L'ensemble 1 représenté à la figure 1 comporte un récipient 2 contenant la poudre libre P selon l'invention, et un applicateur 3 capable de se loger à l'intérieur du récipient lorsque celui-ci est fermé par un capot de fermeture 4. L'applicateur 3 comporte une partie de préhension 6 logeant une source vibrante 8 comportant une source d'énergie 7 et un interrupteur 9 de marche-arrêt. La source vibrante 8 comporte dans cet exemple un moteur électrique entraînant en rotation une masselotte, mais la source vibrante peut être réalisée de toute autre manière. La surface d'application 15 de l'applicateur 3 est par exemple définie par une peau 16 qui peut recouvrir comme illustré une mousse 17. Le moteur et la masselotte sont contenus dans un logement 18 de l'applicateur.

Le récipient 2 peut éventuellement comporter, une paroi ajourée séparant le compartiment contenant la poudre libre P de celui destiné au prélèvement de la composition par la surface d'application, comme illustré sur la figure 2 et décrit dans la demande WO 2006/090343, dont le contenu est incorporé par référence.

Dans la variante de réalisation illustrée à la figure 2, le dispositif comporte une source vibrante 180, qui peut être ou non fixée de manière amovible sur un applicateur 181 portant un élément d'application 182 constitué par exemple par une mousse.

La mise en marche de la source vibrante peut être commandée par un interrupteur 183 prévu sur une face d'extrémité de l'applicateur.

Dans l'exemple de la figure 3, l'élément d'application 182 est chargé en produit P à travers une paroi ajourée 186 séparant un logement 187 recevant l'élément d'application lorsque l'applicateur ferme le récipient et un espace 188 contenant la réserve de produit. L'applicateur est par exemple fixé sur le récipient 190 par vissage.

Dans l'exemple de cette figure, le produit P est contenu dans une coupelle 193 logée dans un récipient 190 sur lequel peut se fixer l'applicateur 181.

Ce dernier comporte, par exemple, un élément d'application 182 qui s'engage à l'intérieur du récipient lorsque l'applicateur est en place sur celui-ci. L'élément d'application comporte, par exemple, une mousse.

L'élément d'application 182 vient au contact du produit P présent dans la coupelle 193, celle-ci étant pressée contre l'élément d'application 182 par un organe de rappel élastique, par exemple un ressort 191, s'interposant entre la coupelle 193 et le fond du récipient 190. Lorsque la source vibrante 180 est mise en marche par exemple en s'appuyant sur l'interrupteur 183, les vibrations qui se transmettent à l'élément d'application 182 permettant de déliter le produit P et de charger l'élément d'application 182.

L'applicateur peut comporter tout élément d'application, par exemple une brosse, un pinceau, un embout floqué, un fritté ou une lingette.

Dans une autre variante non illustrée, l'applicateur comporte le récipient contenant la poudre à appliquer et une source vibrante solidaire du récipient lors de l'utilisation de celui-ci. Le récipient comporte par exemple un col sur lequel est montée une pièce de support d'un organe d'application, par exemple une mousse suffisamment poreuse pour permettre la sortie de la poudre. Dans une variante non illustrée, la source vibrante appartient à une unité amovible lui permettant d'être réutilisée sur un autre récipient.

L'applicateur peut comporter tout élément d'application, par exemple une brosse, un pinceau, un embout floqué, un fritté ou une lingette. Dans une variante non illustrée, on monte sur le doigt de l'utilisateur une source vibrante, comme décrit dans la demande FR 2 904 923. La surface d'application peut être définie par l'extrémité du doigt qui vient en contact avec le produit pour le prélever et l'appliquer, ou en variante, par un patin applicateur fixé sur le doigt.

### Poudre libre

### Liant

Une poudre libre de l'invention contient au moins un liant. Le liant peut être constitué de composés hydrocarbonés et/ou siliconé et/ou fluorés.

Par « liant », on entend au sens de l'invention une phase grasse qui peut comprendre une huile et/ou un composé pâteux et/ou un composé cireux.

Ces composés peuvent être hydrocarbonés et/ou siliconés et/ou fluorés.

De préférence, le liant comprend au moins un dérivé siliconé. Le terme dérivé siliconé englobe l'ensemble des composés contenant du silicone et appartenant respectivement à la famille des huiles siliconées, des résines siliconées, des cires siliconées, des gommes siliconées et à la famille des élastomères siliconés.

Des exemples de silicones de chacune de ces familles sont décrits dans la demande FR 2 881 644.

Le liant peut ainsi contenir une teneur en silicone allant de 10 à 100 % en poids.

A titre d'exemples de composés hydrocarbonés, on peut citer :
- les huiles hydrocarbonées d'origine animale telles que le perhydrosqualène,
- les huiles hydrocarbonées végétales telles que les triglycérides liquides d'acides gras de 4 à 10 atomes de carbone comme les triglycerides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité,
- les hydrocarbures linéaires ou ramifiés, d'origine minérale ou synthétique tels que la vaseline, les polydécènes, le polyisobutène hydrogéné tel que le parléam,
- les esters et les éthers de synthèse comme les huiles de formule R1COOR2 dans laquelle R1 représente le reste d'un acide gras supérieur comportant de 6 à 29 atomes de carbone et R2 représente une chaîne hydrocarbonée contenant de 3 à 30 atomes de carbone, telles que l'huile de Purcellin, le myristate d'isopropyle, le palmitate d'éthyl-2 hexyle, le stéarate d'octyl-2 dodécyle, l'érucate d'octyl-2 dodécyle, l'isostéarate d'isostéaryle, le propionate d'arachidyle, le benzoate d'octyl-2 dodecyle; les esters hydroxylés comme l'isostéaryl lactate, l'octylhydroxystéarate, l'hydroxystéarate d'octyldodécyle, le diisostéarylmalate, le citrate de triisocétyle ; les esters de polyol comme le dioctanoate de propylène glycol, le diheptanoate de néopentylglycol, le diisononanoate de diéthylèneglycol et les esters du pentaérythritol ,
- les alcools gras liquides à température ambiante à chaîne carbonée ramifiée et/ou insaturée ayant de 12 à 24 atomes de carbonne comme l'octyldodécanol, le 2-hexyldécanol, le 2-butyloctanol, le 2-undécylantadécanol, l'alcool isostéarylique oléique.

A titre d'exemples de composés siliconés, on peut citer :
- Les polysiloxanes linéaires, dont le degré de polymérisation est de préférence de 6 à 2000 environ. On peut citer, par exemple: les polydiméthylsiloxanes (PDMS) de viscosité supérieure à 10 mPa.s, les phényldiméthicones, les phényltriméthicones, les polyphénylméthyl-siloxanes et leurs mélanges ;
- les cires de silicone comme des polysiloxanes linéaires substitués. On peut citer, par exemple, les cires de silicone polyéther, les alkyl ou alkoxy-diméthicones ayant de 16 a 45 atomes de carbone,
- les gommes de silicone comme des polysiloxanes de masse moléculaire élevée, de l'ordre de 200 000 a 1 000 000 et ayant une viscosité supérieure à 500 000 mPa.s. Elles peuvent être utilisées seules ou en mélange avec un solvant tel qu'une huile polydiméthylsiloxane ou polyphénylsiloxane ;
- les résines de silicone ayant pour formule générale :

   [(RR'R")3SiO1/2]x[SiO4/2]y
dans laquelle R, R' et R" représentent indépendamment une chaine alkyle linéaire ou ramifiée de 1 à 10 carbone, ou un radical phenyl, et x et y sont tels que le ratio (RR'R")3SiO1/2 / SiO4/2 est compris de 0,5/1 à 1,5/1 ; lesdites résines de silicone peuvent être utilisées seules ou véhiculées avec un solvant tel que les silicones cycliques ;
- les silicones phénylées comme les phényltrimethicones, les diphenyldimethicones, les phenyl dimethicones, les diphényl méthyldiphényl trisiloxanes les phényl triméthylsiloxy diphénylsiloxanes.

A titre d'exemples de composés fluorés, on peut citer :
- les silicones fluorées, les perfluoropolyéthers.

Selon un mode particulier de l'invention, le liant est siliconé.

Dans tous les cas, les poudres libres de l'invention sont caractérisées par un taux de liant allant de 0,1 à 7% en poids, de préférence de 1 à 6% et de manière encore plus préférentielle de 2 à 5% par rapport au poids total de la composition.

### Matériau particulaire lamellaire

L'intérêt de la forme lamellaire est de favoriser l'étalement de la poudre avec l'applicateur vibrant.

La poudre libre contient en plus du liant une phase pulvérulente, au moins partiellement composée de matériau(x) particulaire(s) lamellaire(s).

Par matériau particulaire lamellaire, on entend un matériau particulaire dont l'une des dimensions est très inférieure aux deux autres. De tels matériaux particulaires lamellaires comportent le plus souvent une épaisseur E bien inférieure à leur longueur L1 ou largeur L2.

De préférence, le rapport E/L1 et E/L2 est inférieur ou égal à 0,5, de préférence inférieur ou égal à 0,3, de préférence inférieur ou égal 0,1.

L'épaisseur E moyenne des matériaux particulaires lamellaires peut aller de 0,1 à 5 microns (µm) et de préférence de 0,2 à 3 microns (µm).

La longueur L1 moyenne des matériaux particulaires lamellaires peut aller de 1 à 200 microns (µm) et de préférence de 2 à 70 microns (µm).

La largeur L2 moyenne des matériaux particulaires lamellaires peut aller de 1 à 200 m et de préférence de 2 à 70 microns (µm).

Le matériau particulaire lamellaire utilisé selon l'invention peut être minéral ou organique.

Il peut être non coloré, étant utilisé à titre de charge ou coloré pour apporter un effet coloriel, étant utilisé à titre de pigments, par exemple de nacres.

Comme exemples de matériaux particulaires lamellaires, sont inclus des charges, des pigments, notamment minéraux, des nacres. On peut utiliser des charges lamellaires et/ou des nacres lamellaires.

A titre d'exemples de charges lamellaires convenant à l'invention, on peut citer le talc, le mica, le sulfate de baryum, le kaolin, le carbonate de calcium précipité, le carbonate et l'hydro-carbonate de magnésium, l'hydroxyapatite, la lauroyl lysine, le verre, des particules de polytétrafluoroéthylène (PTFE) (comme Ceridust 9205 F de Clariant, ou Fluoropure 103 C de Shamrock Technologies), le nitrure de bore (Ceram Blanche 1 et Ceram Blanche par la société SPCI et PUHP par la société Saint-Gobain Ceramics), la silice lamellaire (SG Flake 3M des sociétés Maprecos et Chemicelen de Sumitomo), et leurs mélanges.

A titre d'exemples de nacres lamellaires convenant à l'invention, on peut citer notamment l'oxychlorure de bismuth, le mica recouvert de titane ou d'oxychlorure de bismuth (Biron LF 2000 de Merck), la poudre d'oxychlorure de bismuth et d'oxyde de zinc (comme Pearl II UCR de Farmaquimia), et leurs mélanges.

Comme exemples de pigments minéraux sous forme lamellaire utilisables selon l'invention, on peut citer notamment les oxydes de zirconium, les oxydes d'aluminium, et leur mélange.

Comme autres nacres susceptibles d'être sous forme lamellaire et utilisables selon l'invention, on peut citer notamment le mica recouvert de titane, ou d'oxychlorure de bismuth, ou avec des oxydes de fer, ou avec un pigment organique du type précité, ainsi que les nacres à base d'oxychlorure de bismuth.

De façon avantageuse, on utilise selon l'invention des matériaux particulaires lamellaires présentant un indice de réfraction supérieur ou égal à 1,7, susceptibles de conférer une bonne couvrance après application de la composition sur la peau. En particulier, on utilisera des matériaux particulaires lamellaires présentant un indice de réfraction allant de 1,7 à 2,2. De telles matériaux particulaires lamellaires sont notamment le nitrure de bore, l'oxychlorure de bismuth, l'oxyde d'aluminium, l'oxyde de zirconium et leurs mélanges.

Selon un mode préféré de mise en oeuvre de l'invention, les matériaux particulaires lamellaires sont choisis parmi l'oxychlorure de bismuth, le nitrure de bore, et leurs mélanges.

La teneur en matériaux particulaires lamellaires selon l'invention sera choisie en fonction du résultat recherché selon l'invention, et notamment en vue d'obtenir un produit présentant une bonne couvrance et qui soit facile à appliquer en association avec l'applicateur vibrant.

En particulier, la teneur en matériau particulaire lamellaire selon l'invention peut représenter notamment de 5 à 90% en poids, de préférence de 10 à 80% en poids, de préférence encore de 20 à 60% en poids, par rapport au poids total de la composition.

A titre d'exemple, on utilisera de l'oxychlorure de bismuth et/ou du nitrure de bore en une teneur, pour chacun d'eux, allant de 2 à 50% en poids et de préférence de 5 à 40% en poids par rapport au poids total de ladite composition.

### Matériau particulaire non lamellaires

La phase pulvérulente de la composition selon l'invention peut comporter en outre des matériaux particulaires non lamellaires, de natures minérales ou organiques de toute forme, quelle que soit la forme cristallographique (par exemple sphérique, cubique, hexagonale, orthorombique, etc).

Ces matériaux particulaires non lamellaires peuvent être des charges et/ou des pigments.

On peut citer notamment des charges non lamellaires telles que la silice non lamellaire, les poudres de polyamide (Nylon®) , de poly-β-alanine et de polyéthylène, l'amidon, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, les poudres de polyuréthanes, les charges composites, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Comme pigments non lamellaires, on peut citer notamment les pigments choisis parmi le dioxyde de titane, éventuellement traité en surface, les oxydes de cérium, ainsi que les oxydes de zinc, de magnésium, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre, et leurs mélanges.

Selon un mode particulier de l'invention, la composition comprendra, outre un ou plusieurs matériaux particulaires lamellaires tels que décrits précédemment, au moins un matériau particulaire non lamellaire de forme sphérique. En particulier, le ou les matériaux particulaires de forme sphérique sera(ont) choisie(s) parmi les pigments minéraux et de préférence les oxydes de fer, l'oxyde de titane, l'oxyde de zinc, et leurs mélanges.

Selon un mode particulier, la proportion massique en pigments, notamment minéraux dans ladite composition de l'invention, est supérieure ou égale à 1 %.

Dans le cas où la phase pulvérulente comprend en outre des matériaux particulaires non lamellaires, la teneur en matériaux particulaires lamellaires selon l'invention représentera notamment de 10 à 100% de préférence de 20 à 80% en poids par rapport au poids total desdits matériaux particulaires.
Selon un mode particulier de l'invention, dans le cas où la phase pulvérulente comprend des matériaux particulaires lamellaires et des matériaux particulaires non lamellaires, la proportion des unes par rapport aux autres sera avantageusement de 60-70% de matériaux particulaires lamellaires pour 30-40% de matériaux particulaires non lamellaires en poids par rapport au poids total desdits matériaux particulaires.

### Autres matières colorantes

La composition peut comporter en outre au moins une autre matière colorante, telle que des pigments organiques, un matériau à effet optique spécifique et/ou des colorants liposolubles.

Parmi les pigments organiques, on peut citer le noir de carbone, les pigments de type D & C, et les laques, notamment les laques à base de carmin de cochenille, de baryum, strontium, calcium, aluminium.

Un matériau à effet optique spécifique peut être choisi parmi les particules à reflet métallique, les agents de coloration goniochromatiques, les pigments diffractants, les agents thermochromes, les agents azurants optiques, ainsi que les fibres, notamment, interférentielles.

Les colorants liposolubles sont, par exemple, le rouge Soudan, le DC Red 17, le DC Green 6, le β-carotène, l'huile de soja, le brun Soudan, le DC Yellow 11, le DC Violet 2, le DC orange 5 et le jaune quinoléine.

Les pigments additionnels peuvent être présents dans la composition en une teneur allant de 0,01 à 60% en poids, par rapport au poids total de la composition, de préférence allant de 0,5 à 55% en poids, et préférentiellement allant de 1 à 45% en poids.

Les colorants liposolubles peuvent être présents dans la composition en une teneur allant de 0,0001 % à 5 % en poids, par rapport au poids total de la composition, de préférence allant de 0,001 % à 3 % en poids, et préférentiellement allant de 0,01 % à 2 % en poids.

### Actifs

La poudre libre peut comporter en outre au moins un actif cosmétique ou dermatologique, voire ne pas comporter d'actif dermatologique. Comme actifs cosmétiques, dermatologiques, hygiéniques ou pharmaceutiques, on peut citer les hydratants (polyol comme glycérine), vitamines (C, A, E, F, B, ou PP), acides gras essentiels, huiles essentielles, céramides, sphingolipides, filtres solaires liposolubles ou sous forme de particules de petite taille (<1µm), les actifs spécifiques de traitement de la peau (agents de protection, anti-bactériens, anti-rides...), autobronzants. Ces actifs peuvent être utilisés par exemple à des concentrations de 0 à 10 % et notamment de 0,001 à 5 %, par rapport au poids total de la composition.

La poudre libre peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

La poudre libre peut comprendre, selon le type d'application envisagée, les constituants classiquement utilisés dans les domaines considérés, qui sont présents en une quantité appropriée à la forme galénique souhaitée.

La composition de soin selon l'invention peut être une poudre teintée pour le visage ou pour le corps.

La composition de maquillage des matières kératiniques selon l'invention peut être notamment une composition de maquillage de la peau telle qu'un fond de teint notamment à appliquer sur le visage ou le cou, un produit anti-cernes, un correcteur de teint, un fard à joues, une composition de maquillage pour le corps.

Avantageusement, il s'agira d'une composition de maquillage de la peau, en particulier de la peau du visage, notamment un fond de teint.

### EXEMPLES

### Exemple 1 : Application d'une poudre libre avec un applicateur vibrant

### Exemple de poudre libre

| | | % massique |
|---|---|---|
| A1 | Oxyde de fer jaune | 4,00 |
| | Oxyde de fer rouge | 1,30 |
| | Oxyde de fer noir | 0,36 |
| | Dioxyde de titane | 20,00 |
| | Oxyde de zinc vendu sous la référence Z Cote par la société BASF | 20,00 |
| | **Oxychlorure de bismuth¹** | **30,00** |
| | **Nitrure de bore²** | **6,00** |
| | **Talc³** | **13,74** |
| | Cire de carnauba vendu sous la référence microcare 350 par la société Micropowders | 1,00 |
| A2 | Poly methyl cetyl dimethylsiloxane vendu sous la référence ABILWAX 9801 par la société Goldschmidt | 0,37 |
| | PDMS+Trimethyl siloxy silicate vendu sous la référence DC 593 par la société Dow Corning | 0,59 |
| | PDMS (10cSt) | 1,89 |
| | Tri isocetyl citrate | 0,15 |
| | Conservateur | 0,60 |
| | TOTAL | 100% |

| | | |
|---|---|---|
| 1 : Oxychlorure de bismuth vendu sous la référence BIRON LF 2000 par la société Merck. 2 : Nitrure de bore vendu sous la référence PUHP 3008 par la société Saint-Gobain Ceramics 3 : Talc vendu sous la référence Luzenac 00 par la société Luzenac. | | |

### Mode opératoire

On a mélangé tous les constituants de la phase A1 dans un mélangeur Lodige pendant 15 minutes (sans vidange).

Sous agitation on a ajouté les constituants de la phase A2 dans A1 et on a mélangé pendant 15 minutes.

On a broyé ensuite le mélange obtenu dans un broyeur à broches à une vitesse de 1800 tours/min.

On a tamisé le mélange obtenu sur un tamis de 400 microns.

### Maquillage

On a prélevé la poudre libre avec un applicateur tel que décrit sur la figure 1 sans vibration, puis on a appliqué la poudre par demi-visage.

Sur la première moitié du visage, on a utilisé l'applicateur en mode vibrant à 7000 tr/mn (117 Hz), alors que pour l'autre moitié on a utilisé le même applicateur sans vibration.

On constate que la vibration a facilité l'application, qui est plus rapide. Le maquillage obtenu avec vibration est plus lisse, plus homogène, plus uniforme et moins poudreux.

Cet effet positif de la vibration est particulièrement visible pour les femmes qui ont une peau avec des pores dilatés.

### Exemple 2 : Evaluation de l'impact de la forme des matériaux particulaires

Des maquillages comparatifs ont été réalisés sur un panel de 6 personnes utilisatrices de fond de teint avec deux formules comprenant respectivement des matériaux particulaires lamellaires pour la formule 1 (selon l'invention) ou de matériaux particulaires sphériques pour la formule 2 (comparative).

Lesdites compositions sont préparées comme décrit précédemment à l'exemple 1.

| **Phase** | **ingrédients** | **Formule 1** | **Formule 2** |
|---|---|---|---|
| **A1** | **talc** | **14,89** | **14,89** |
| **A2** | Oxyde de fer jaune | 3,47 | 3,47 |
| | Oxyde de fer rouge | 1,00 | 1,00 |
| | Oxyde de fer noir | 0,34 | 0,34 |
| **A3** | **Oxychlorure de bismuth** | **30,00** | |
| | **Nitrure de bore** | **6,00** | |
| | **Microbilles de résine méthylsilsesquioxane (granulométrie 4.5µm) vendues sous la dénomination Tospearl par GE Toshiba Silicones** | | **36,00** |
| | Dioxyde de titane | 20,00 | 20,00 |
| | Oxyde de zinc | 20,00 | 20,00 |
| | Cire de carnauba vendu sous la référence microcare 350 par la société Micropowders | 1,00 | 1,00 |
| **B** | Tri isocetyl citrate | 0,1650 | 0,1650 |
| | PDMS (10cSt) | 2,0757 | 2,0757 |
| | PDMS+Trimethyl siloxy silicate vendu sous la référence DC 593 par la société Dow Corning | 0,6534 | 0,6534 |
| | CETYL DIMETHICONE | 0,4059 | 0,4059 |
| | | 100,00 | 100,00 |

### Protocole de maquillage

0,06g de chaque formule sont appliqués en hémi-visage sur un panel de 6 modèles (personnes utilisatrices de fond de teint), à l'aide d'un applicateur vibrant. L'application est libre : chaque modèle prélève ladite composition (formule 1) avec un applicateur tel que décrit sur la figure 1 sans vibration, puis l'applique avec vibration sur une moitié du visage. De la même façon, chaque modèle prélève la composition (formule 2 comparative) avec un applicateur tel que décrit sur la figure 1 sans vibration et l'applique avec vibration sur l'autre moitié du visage. Le temps d'application n'a pas été imposé afin de permettre aux modèles d'optimiser le maquillage pour les différentes formules ; le temps d'application moyen est d'environ 1 min pour l'application des 2 formules, chacune en hémi-visage.

Chaque modèle a évalué l'application des deux compositions en termes d'homogénéité du maquillage, de couvrance, et de matité.

Pour chacun des critères évalués, les 6 modèles ont noté plus favorablement la formule 1 (selon l'invention) que la formule (2) :

| Formules testées | | Homogénéité | Couvrance | Matité | Effet poudré |
|---|---|---|---|---|---|
| Formule (matériaux particulaires lamellaires) | 1 | +++ | ++++ | ++++ | ++ |
| Formule (particules sphériques) | 2 | ++ | ++ | +++ | ++++ |

Ces résultats montrent qu'à l'application, le maquillage avec les matériaux particulaires lamellaires (formule 1 selon l'invention) est plus homogène et uniforme que le maquillage avec les matériaux particulaires sphériques. De plus le résultat maquillage tout en étant plus couvrant et plus mat, apporte en outre un effet moins poudré dans le cas de la formule 1 selon l'invention, et donc plus lisse.

### Exemple 3 : Evaluation de l'impact de la nature du liant

On évalue l'impact de la nature du liant sur l'application et le maquillage, à l'aide d'un applicateur vibrant, de deux compositions sous forme de poudre libre contenant respectivement un liant siliconé (formule 3) ou un liant hydrocarboné (formule 4).

| **Phase** | **Nom INCI** | **Formule 1** | **Formule 3** |
|---|---|---|---|
| **A1** | Oxyde de fer jaune | 14,89 | 14,89 |
| **A2** | Oxyde de fer rouge | 3,47 | 3,47 |
| | Oxyde de fer jaune | 1,00 | 1,00 |
| | Oxyde de fer noir | 0,34 | 0,34 |
| **A3** | Nitrure de bore | 30,00 | 30,00 |
| | Oxychlorure de bismuth | 6,00 | 6,00 |
| | Dioxyde de titane | 20,00 | 20,00 |
| | Oxyde de zinc | 20,00 | 20,00 |
| | Cire de carnauba vendu sous la référence microcare 350 par la société Micropowders | 1,00 | 1,00 |
| **B** | **ISOCETYL STEARATE** | | **3,30** |
| | **Tri isocetyl citrate** | **0,1650** | |
| | **PDMS (10cSt)** | **2,0757** | |
| | **PDMS+Trimethyl siloxy silicate vendu sous la référence DC 593 par la société Dow Corning** | **0,6534** | |
| | **CETYL DIMETHICONE** | **0,4059** | |
| | | 100,00 | 100,00 |

Le protocole d'application et de maquillage desdites compositions sur un panel de 6 modèles en hémi-visage, et à l'aide d'un applicateur vibrant, est le même que celui décrit à l'exemple 2.

Outre les critères d'homogénéité, de couvrance et de matité, qui ont été appréciés de façon égale pour les deux compositions (+++), la poudre contenant le liant siliconé, appliquée avec l'applicateur vibrant, donne un résultat encore amélioré, en particulier moins poudré et le relief est moins marqué.

L'ensemble de ces résultats montre que l'association de matériaux particulaires lamellaires et d'un liant siliconé dans la formule poudre appliquée avec un applicateur vibrant permet une application et un résultat maquillage optimum. L'application est plus homogène, le résultat maquillage est plus couvrant, plus mat mais sans effet poudré. De plus le relief est moins marqué. Ce résultat de couvrance sans effet poudré ni marquage du relief est particulièrement intéressant, eu égard aux difficultés à l'obtenir par les méthodes classiques.

L'expression « comportant un » est synonyme de « comportant au moins un » et « compris entre » doit s'entendre bornes incluses.

L'invention n'est pas limitée aux applicateurs illustrés.

## Revendications

1. Procédé de maquillage et/ou de soin de la peau, notamment pour lisser le microrelief cutané, comportant les étapes consistant à :
- appliquer une composition cosmétique sous forme de poudre libre au moyen d'un applicateur vibrant, la composition comportant :
- un matériau particulaire lamellaire en une proportion massique supérieure ou égale à 5 % par rapport au poids total de la composition,
- entre 0,1 et 7 % en masse d'un liant hydrocarboné, siliconé et/ou fluoré.

2. Procédé selon la revendication 1, comportant l'étape consistant à prélever la poudre sans soumettre l'applicateur à des vibrations, en amenant l'applicateur au contact de la composition contenue dans un récipient.

3. Procédé selon la revendication 1 ou 2, comportant l'étape consistant à soumettre l'applicateur à des vibrations après le prélèvement, alors que l'applicateur est encore au-dessus du récipient, de façon à éliminer un éventuel surplus de composition sur l'applicateur.

4. Procédé selon l'une quelconque des revendications précédentes, la poudre étant appliquée sur la peau nue.

5. Procédé selon l'une quelconque des revendications précédentes, le matériau particulaire lamellaire étant choisi parmi des charges, des nacres et leurs mélanges.

6. Procédé selon l'une quelconque des revendications précédentes, le matériau particulaire lamellaire étant choisi parmi le talc, le mica, le sulfate de baryum, le kaolin, le carbonate de calcium précipité, le carbonate et l'hydro-carbonate de magnésium, l'hydroxyapatite, des particules de polytétrafluoroéthylène (PTFE), l'oxychlorure de bismuth, le mica recouvert de titane ou d'oxychlorure de bismuth, la poudre d'oxychlorure de bismuth et d'oxyde de zinc, le nitrure de bore, la silice lamellaire, l'oxyde d'aluminium, l'oxyde de zirconium et leurs mélanges.

7. Procédé selon l'une quelconque des revendications 1 à 6, le matériau particulaire lamellaire comportant au moins de l'oxychlorure de bismuth, du nitrure de bore ou leur mélange.

8. Procédé selon l'une quelconque des revendications précédentes, la composition comportant en outre au moins un matériau particulaire non lamellaire.

9. Procédé selon la revendication précédente, le matériau particulaire non lamellaire étant choisi parmi les pigments, notamment minéraux.

10. Procédé selon la revendication 9, la proportion massique en pigments, notamment minéraux, étant supérieure ou égale à 1 %.

11. Procédé selon l'une quelconque des revendications 1 à 10, le liant étant choisi parmi les liants hydrocarboné, siliconé et/ou fluoré et notamment :
- les huiles hydrocarbonées d'origine animale telles que le perhydrosqualène,
- les huiles hydrocarbonées végétales telles que les triglycérides liquides d'acides gras de 4 à 10 atomes de carbone comme les triglycerides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité,
- les hydrocarbures linéaires ou ramifiés, d'origine minérale ou synthétique tels que la vaseline, les polydécènes, le polyisobutène hydrogéné tel que le parléam,
- les esters et les éthers de synthèse comme les huiles de formule R1COOR2 dans laquelle R1 représente le reste d'un acide gras supérieur comportant de 6 à 29 atomes de carbone et R2 représente une chaîne hydrocarbonée contenant de 3 à 30 atomes de carbone, telles que l'huile de Purcellin, le myristate d'isopropyle, le palmitate d'éthyl-2 hexyle, le stéarate d'octyl-2 dodécyle, l'érucate d'octyl-2 dodécyle, l'isostéarate d'isostéaryle, le propionate d'arachidyle, le benzoate d'octyl-2 dodecyle; les esters hydroxylés comme l'isostéaryl lactate, l'octylhydroxystéarate, l'hydroxystéarate d'octyldodécyle, le diisostéarylmalate, le citrate de triisocétyle ; les esters de polyol comme le dioctanoate de propylène glycol, le diheptanoate de néopentylglycol, le diisononanoate de diéthylèneglycol et les esters du pentaérythritol ,
- les alcools gras liquides à température ambiante à chaîne carbonée ramifiée et/ou insaturée ayant de 12 à 24 atomes de carbonne comme l'octyldodécanol, le 2-hexyldécanol, le 2-butyloctanol, le 2-undécylantadécanol, l'alcool isostéarylique oléique,
- les polysiloxanes linéaires, dont le degré de polymérisation est de préférence de 6 à 2000 environ, notamment les polydiméthylsiloxanes (PDMS) de viscosité supérieure à 10 mPa.s, les phényldiméthicones, les phényltriméthicones, les polyphénylméthyl-siloxanes et leurs mélanges,
- les cires de silicone comme des polysiloxanes linéaires substitués, les cires de silicone polyéther, les alkyl ou alkoxy-diméthicones ayant de 16 a 45 atomes de carbone,
- les gommes de silicone comme des polysiloxanes de masse moléculaire élevée, de l'ordre de 200 000 a 1 000 000 et ayant une viscosité supérieure à 500 000 mPa.s, utilisées seules ou en mélange avec un solvant tel qu'une huile polydiméthylsiloxane ou polyphénylsiloxane,
- les résines de silicone ayant pour formule générale :
[(RR'R")3SiO1/2]x[SiO4/2]y
dans laquelle R, R' et R" représentent indépendamment une chaine alkyle linéaire ou ramifiée de 1 à 10 carbone, ou un radical phenyl, et x et y sont tels que le ratio (RR'R")3SiO1/2 / SiO4/2 est compris de 0,5/1 à 1,5/1,
- les silicones phénylées comme les phényltrimethicones, les diphenyldimethicones, les phenyl dimethicones, les diphényl méthyldiphényl trisiloxanes les phényl triméthylsiloxy diphénylsiloxanes,
- les silicones fluorées, les perfluoropolyéthers et les alcanes perfluorés.

12. Ensemble (1) cosmétique, comportant :
- un récipient (2) contenant une poudre libre cosmétique, comportant :
- un matériau particulaire lamellaire en une proportion supérieure ou égale à 5 % en poids par rapport au poids total de la composition,
- un liant hydrocarboné, siliconé et/ou fluoré,
- un applicateur (3),
- une source vibrante (8) pour soumettre l'applicateur à des vibrations au moins au moment de l'application de la composition sur la peau et/ou du prélèvement de la composition.

13. Ensemble selon la revendication précédente, l'applicateur comportant une mousse, un pinceau, un flocage, un tissé ou non tissé, définissant une surface d'application destinée à venir au contact de la peau.

14. Ensemble selon l'une quelconque des revendications 12 à 13, le matériau particulaire lamellaire comportant de l'oxychlorure de bismuth et/ou du nitrure de bore, la teneur massique dans la composition en oxychlorure de bismuth et/ou nitrure de bore allant de 5à 90 % en poids, mieux de 20 à 60 % en poids.

15. Ensemble selon l'une quelconque des revendications 12 à 14, le liant étant siliconé.
